# EUROPÄISCHE PATENTSCHRIFT

(11) **EP 3 863 570 B1**
(45) Veröffentlichungstag und Bekanntmachung des Hinweises auf die Patenterteilung: **03.04.2024**
(21) Anmeldenummer: 19786737.7
(22) Anmeldetag: 04.10.2019
(51) Int. Cl.: A61F 2/50, A61F 2/76, A61F 2/78, G01B 11/16, G01B 15/06, G01B 17/04

(54) **HÜLLKÖRPER UND VERFAHREN ZUM ERFASSEN EINER KONTUR EINES AMPUTATIONSSTUMPFES**
ENVELOPING BODY AND METHOD FOR DETECTING A CONTOUR OF AN AMPUTATION STUMP
VOLUME ENGLOBANT ET PROCÉDÉ DE DÉTECTION D'UN CONTOUR D'UN MOIGNON D'AMPUTATION

(30) Priorität: 11.10.2018 DE 102018125144
(43) Veröffentlichungstag der Anmeldung: 18.08.2021
(60) Teilanmeldung: 24159493.6
(73) Patentinhaber: Ottobock SE & Co. KGaA, 37115 Duderstadt (DE)
(72) Erfinder: ALBRECHT-LAATSCH, Erik, 37115 Duderstadt (DE); NOLTE, Michael, 37115 Duderstadt (DE); DRIESEBERG, Jens, 37115 Duderstadt (DE); WILL, Christian, 37115 Duderstadt (DE); BETZ, Sebastian, 37115 Duderstadt (DE); ENGELBART, Hendryk, 37115 Duderstadt (DE); FINKE, Lars Benjamin, 37115 Duderstadt (DE); BIELEFELD, Thilo, 37115 Duderstadt (DE)
(74) Vertreter: Gramm, Lins & Partner Patent- und Rechtsanwälte PartGmbB
(86) Internationale Anmeldenummer: PCT/EP2019/076862
(87) Internationale Veröffentlichungsnummer: WO 2020/074374

(56) Entgegenhaltungen:
- WO-A1-2014/130878
- WO-A1-2016/033469
- WO-A1-2017/011753

## Beschreibung

Die Erfindung betrifft einen Hüllkörper zum zumindest teilweisen Erfassen einer Kontur einer Gliedmaße, wobei der Hüllkörper einen Grundkörper aufweist. Die Erfindung betrifft zudem ein Verfahren zum zumindest teilweisen Erfassen einer Kontur einer Gliedmaße, bei dem ein derartiger Hüllkörper zum Einsatz kommt.

Unter einem Hüllkörper werden insbesondere Prothesenliner und Bandagen verstanden, die eine Gliedmaße oder einen Amputationsstumpf zumindest teilweise, vorzugsweise jedoch vollständig, umschließen, wenn sie bestimmungsgemäß angelegt wurden. Die Gliedmaßen können eine obere Gliedmaße, also ein Arm, oder eine untere Gliedmaße, also ein Bein, aber auch ein Teil eines Rumpfes des Trägers sein.

Eine Prothese verfügt in der Regel über einen Prothesenschaft, in den der Amputationsstumpf eingeführt wird. Der Prothesenschaft besteht in der Regel aus einem starren Material, beispielsweise einem Kohlefaserverbundwerkstoff. Insbesondere bei Beinprothesen, mit denen der Patient geht oder läuft wirken dabei große Kräfte sowohl auf den Prothesenschaft als auch auf den Amputationsstumpf. Um zu vermeiden, dass es dabei zu schmerzhaften Druckstellen kommt ist es von großer Wichtigkeit, den Prothesenschaft, der in der Regel individuell für den Träger der Prothese angefertigt wird, möglichst gut auf die Form des Amputationsstumpfes anzupassen. Dabei besteht jedoch die Schwierigkeit, dass der Amputationsstumpf sowohl über knochige Anteile verfügt, die besonders druckempfindlich sind und daher geschützt werden müssen und Weichteile aufweist, die sich unter der Belastung beim Gehen oder Stehen verformen. Aus diesem Grund ist es sinnvoll, zum zumindest teilweisen Erfassen der Form und Kontur des zu fertigenden Prothesenschaftes den Amputationsstumpf zu komprimieren, also insbesondere mit einem Druck zu beaufschlagen, und mit einer Zweckform zu modifizieren. Man spricht dabei von einer Vorkompression.

Aus dem Stand der Technik sind verschiedene Verfahren bekannt, einen Prothesenschaft zu erzeugen. Die Standardmethode war lange Zeit, den Amputationsstumpf beispielsweise mit Gips abzuformen. Dies hat den Vorteil, dass beispielsweise der Orthopädietechniker, der den Abdruck erstellt, mit seinen Händen Druck auf den Amputationsstumpf ausüben kann, und so die Form des Stumpfes im Sinne einer Zweckform zu manipulieren. Von diesem so erstellten Negativabdruck wird eine Positivform des Amputationsstumpfes erstellt, die dann zur Herstellung des Prothesenschaftes herangezogen wird. Nachteilig dabei ist, dass die Prozedur für den Patienten aufwendig und unangenehm ist, da der Patient so lange warten muss, bis der Gips ausgehärtet ist, und die Gipsform vom Amputationsstumpf abgenommen werden kann.

Die WO 2017/011753 A1 beschreibt ein Verfahren, bei dem unterschiedliche Sensoren verwendet werden. Am Amputationsstumpf werden sowohl eine Mehrzahl von Positionssensoren als auch eine Mehrzahl von Orientierungssensoren angeordnet, deren Messdaten und/oder Position beispielsweise durch optische Sensoren erfasst werden. Dies geschieht nacheinander und hat mehrere Auswertungsschritte zur Folge.

Um während des Abformprozesses eine gleichmäßige Vorkompression des Amputationsstumpfes zu erreichen, sind aus dem Stand der Technik unterschiedliche Verfahren bekannt. So wird beispielsweise vorgeschlagen, den Amputationsstumpf in einen Behälter einzuführen, der mit Sand verfüllt wird. Auf diese Weise kann durch den Patienten selbst ein Druck aufgebracht werden, der dem später beim Stehen entstehenden Druck entspricht. In dem Sand entsteht auf diese Weise ein Negativabdruck des Amputationsstumpfes, der beispielsweise ausgegossen werden kann. Alternativ dazu wird vorgeschlagen, den Amputationsstumpf in einer flexiblen Membran in einen Wassertank einzuführen und den Druck des Wassers zur Vorkompression zu nutzen. Innerhalb des Wassertanks kann der so präparierte Amputationsstumpf beispielsweise berührungslos, durch optisches Scanverfahren, gescannt und auf diese Weise die Kontur abgenommen werden.

Aus der DE 20 2016 001 130 U1 ist hingegen eine Vorrichtung bekannt, die über einen Befestigungsring mit einem daran angeordneten Liner verfügt, in den der Amputationsstumpf eingeführt wird. Der Vorteil dieser Vorrichtung besteht darin, dass durch den Liner, in den der Amputationsstumpf eingeführt wird, eine Druckverteilung auf den Amputationsstumpf ausgeübt wird, die der durch den tatsächlichen Liner, der innerhalb des Prothesenschaftes getragen wird, weitestgehend entspricht. Auch in diesem Liner, der über den Haltering oder Befestigungsring gehalten wird, kann der Amputationsstumpf dann über berührungslose Scanverfahren abgescannt werden.

Aus einer Pressemitteilung des Fraunhofer-Institutes für Silicatforschung ISC aus dem Jahr 2015 ist es prinzipiell bekannt, Dehnungs- oder Drucksensoren aus Elastomeren aus Silicon in Textilien zu integrieren. Die Erklärung ist unter https-//www.isc.fraunhofer.de/de/presse-und-medien/pressearchiv/pressearchiv-2015/textilintegrierte-drucksensoren.html abrufbar.

Nachteilig ist jedoch, dass die Druckverteilung nicht manuell angepasst werden kann, wie dies der Orthopädietechniker beispielsweise bei der Gipsabdruck-Methode tut. Zudem ist das Abscannen des Amputationsstumpfes beispielsweise in einem Wassertank mit hohem apparativem Aufwand verbunden. Der Erfindung liegt daher die Aufgabe zugrunde, einen Liner so weiterzuentwickeln, dass die Nachteile aus dem Stand der Technik behoben oder zumindest gemildert werden.

Die Erfindung löst die gestellt Aufgabe durch einen Hüllkörper gemäß dem Oberbegriff des Anspruchs 1, wobei der Hüllkörper wenigstens einen Sensor aufweist, der eingerichtet ist, Messdaten zu erfassen, aus denen ein Abstand und/oder eine Relativposition zwischen zwei Punkten in
oder an dem Grundkörper ermittelbar ist, der sich dadurch auszeichnet, dass der Hüllkörper ein Prothesenliner ist und der Grundkörper aus einem Linermaterial hergestellt ist. Die erfindungsgemäße Idee besteht folglich darin, den Sensor bereits so auszustatten, dass zumindest ein Teil der Kontur des Hüllkörper von in dem Hüllkörper angeordneten Elementen, in diesem Fall dem wenigstens einen Sensor, ermittelt werden kann oder zumindest entsprechende Messdaten erfasst werden, mit denen dies möglich ist. Dadurch wird der apparative Aufwand zum Erfassen der Kontur deutlich reduziert und zudem das Verfahren beschleunigt, was insbesondere für den Patienten von Vorteil ist.

Bevorzugt verfügt der Sensor über wenigstens einen Sender für eine Messstrahlung und einen Empfänger für die Messstrahlung, die derart angeordnet sind, dass vom Sender ausgesandte Messstrahlung zumindest teilweise vom Empfänger empfangen wird. Auf diese Weise lässt sich beispielsweise über eine Laufzeitmessung, der Abstand zwischen dem Sender und dem Empfänger bestimmen.

Bevorzugt befindet sich dabei am ersten Punkt ein Sender und ein Empfänger befindet, wobei sich vorzugsweise am zweiten Punkt ein Reflektor für die Messstrahlung befindet. Dabei muss der Reflektor kein separates Bauteil beispielsweise in Form eines Spiegels sein, sondern kann auch in Form einer Grenzfläche zwischen zwei verschiedenen Materialien ausgebildet sein. Dies ist beispielsweise bei der Verwendung von Ultraschall als Messstrahlung von Vorteil, die an Grenzflächen reflektiert wird. Eine solche Grenzfläche ist beispielsweise dort vorhanden, wo das Material des Grundkörpers auf die Gliedmaße des Trägers oder Patienten trifft. Je nach verwendeter Messstrahlung zeichnet sich eine Grenzfläche durch einen hinreichend großen Dichtesprung der beiden angrenzenden Materialien, einen hinreichend großen Sprung der optischen Brechungsindizes der angrenzenden Materialien oder einen Sprung einer anderen physikalischen oder chemischen Größe an der Grenzfläche aus.

Alternativ befindet sich vorzugsweise an dem ersten Punkt der Sender des Sensors und an dem zweiten Punkt der Empfänger. Messstrahlung, die vom Sender ausgesandt wird, trifft zumindest teilweise auf den jeweiligen Empfänger. Ist beispielsweise die Intensität der empfangenen Messstrahlung am Empfänger bekannt, wenn sich der Hüllkörper im entspannten Zustand befindet, ohne dass er über eine Gliedmaße, beispielsweise einen Amputationsstumpf gezogen wurde, kann aus der im angelegten Zustand empfangenen Intensität bestimmt werden, inwieweit das Material des Grundkörpers, in dem sich die Messstrahlung bewegt, gedehnt wurde. Je länger der Weg zwischen dem Sender der Messstrahlung und dem Empfänger ist, desto geringer ist die am Empfänger empfangene Intensität der Messstrahlung.

Alternativ oder zusätzlich dazu kann die Laufzeit der Messstrahlung vom Sender zum Empfänger bestimmt werden. Darauf lässt sich die Strecke zwischen Sender und Empfänger bestimmen, sofern die Geschwindigkeit, mit der sich die Messstrahlung ausdehnt, bekannt ist.

Alternativ oder zusätzlich dazu befinden sich Sender und Empfänger am ersten Punkt und am zweiten Punkt befindet sich ein Reflektor für die jeweilige Messstrahlung. Die Funktionsprinzipien der Messung sind dabei identisch, allerdings wird die doppelte Wegstrecke gemessen, wodurch das Signal-zu-Rausch-Verhältnis verbessert wird.

Bevorzugt handelt es sich bei der Messstrahlung um eine elektromagnetische Strahlung, insbesondere sichtbares Licht, Radarstrahlung und/oder Röntgenstrahlung. Insbesondere bei Röntgenstrahlung ist es möglich, die Messstrahlung auch durch die sich im Hüllkörper befindende Gliedmaße hindurch zu senden, auch wenn dies gegebenenfalls gesundheitliche Nachteile für den Patienten aufweist. Alternativ oder zusätzlich dazu kann die Messstrahlung auch eine magnetische Strahlung insbesondere in Form eines magnetischen Wechselfeldes sein. In diesem Fall ist der Sender eine elektromagnetische Spule, also ein zur Spule gewickelter elektrischer Leiter, der mit einem elektrischen Wechselstrom beaufschlagt wird. Dadurch generiert die als Sender wirkende Spule, die auch als Sendespule bezeichnet wird, ein magnetisches Wechselfeld. Der Empfänger beinhaltet dabei vorzugsweise wenigstens eine Empfängerspule, die sich in diesem magnetischen Wechselfeld befindet. Dadurch kommt es in der Empfängerspule zur Induktion eines elektrischen Stroms und/oder einer elektrischen Spannung, die gemessen werden kann und von der Entfernung zwischen der Sendespule und der Empfängerspule abhängt. Zudem hängen die Größe und die Phase der induzierten elektrischen Größe in der Empfängerspule von der Orientierung der Sendespule zur Empfängerspule ab.

Daher ist es von Vorteil, wenn insbesondere als Sender mehrere, vorzugsweise senkrecht aufeinander stehende Spulen verwendet werden. Diese können gleichzeitig mit elektrischem Wechselstrom betrieben werden. Je nach Phasenbeziehung zwischen dem Wechselstrom der einzelnen Spulen lässt sich dabei eine Abstrahlrichtung, in der das magnetische Wechselfeld vornehmlich abgesandt wird, beeinflussen. Es hat sich als vorteilhaft herausgestellt, wenn ein magnetisches Wechselfeld erzeugt wird, dessen vornehmliche Richtung variiert, vorzugsweise zyklisch umläuft. Dies hat zur Folge, dass das in der Empfängerspule induzierte Magnetfeld immer dann minimal ist, wenn die Vorzugsrichtung oder Hauptsenderichtung, in die das Magnetfeld mit der maximalen Intensität gesendet wird, nicht auf die jeweilige Empfängerspule des Empfängers zeigt.

In dieser Ausgestaltung ist es folglich möglich, auch die Orientierung der Empfängerspule relativ zum Sender zu ermitteln. Dazu ist es von Vorteil, wenn die Ausgangsorientierung, also die Orientierung des Senders relativ zum Empfänger im entlasteten Zustand des Hüllkörpers, also ohne Gliedmaße, bekannt ist.

Alternativ oder zusätzlich dazu kann die Messstrahlung auch Schallwellen beinhalten. Dabei bieten sich insbesondere Ultraschallwellen an. Auch in diesem Fall können Sender und Empfänger an zwei unterschiedlichen Orten angeordnet sein, wodurch beispielsweise über Laufzeitmessungen die einfache Strecke, also der Abstand zwischen den beiden Punkten, gemessen wird. Befinden sich Sender und Empfänger der Messstrahlung am gleichen Ort, ist am zweiten Punkt vorzugsweise ein Reflektorelement angeordnet, das die Messstrahlung reflektiert. Das Reflektorelement muss dabei wie bereits ausgeführt kein separates Bauteil sein. Auch bei der Verwendung von Schallwellen als Messstrahlung kann auf diese Weise die doppelte Strecke gemessen und damit das Signal-zu-Rausch-Verhältnis verbessert werden.

Vorteilhafterweise verfügt der Hüllkörper über mehrere Empfänger und/oder mehrere Reflektoren. In einer bevorzugten Ausgestaltung verfügt der Hüllkörper beispielsweise über nur einen Sender für die jeweilige Messstrahlung. Der Hüllkörper verfügt jedoch in diesem Fall über eine Mehrzahl, vorzugsweise wenigstens 10, weiter bevorzugt wenigstens 20, besonders bevorzugt wenigstens 50 oder gar wenigstens 100 Empfänger und/oder Reflektoren für die Messstrahlung. Auf diese Weise können die Abstände vieler Punkte, nämlich der Stellen, an denen sich die Empfänger oder Reflektoren befinden, zum Sender festgestellt und ermittelt werden. Da sich die Anordnung der einzelnen Empfänger oder Reflektoren relativ zueinander, also insbesondere die Nachbarschaftsbeziehungen zwischen den Bauteilen, nicht ändern können, ohne dass der Hüllkörper zerstört wird, lässt sich aus den Abständen zu den unterschiedlichen Punkten die Kontur des Hüllkörper ermitteln. Alternativ oder zusätzlich dazu ist der Sensor eingerichtet, auch eine Richtung, in der sich der jeweilige Empfänger und/oder der jeweilige Reflektor befinden, zu bestimmen. Auf diese Weise ist es noch einfacher, aus den Messdaten die Kontur des Prothesenliners und damit die Kontur der unter dem Druck des Hüllkörpers stehenden Gliedmaße zu bestimmen.

Vorteilhafterweise ist der Sender im distalen Bereich des Hüllkörpers, also dem geschlossenen unteren Ende eines Prothesenliners, angeordnet.

Vorzugsweise verfügt der wenigstens eine Sensor über einen Dehnungssensor, mit dem ein Abstand zwischen zwei Punkten bestimmt werden kann, zwischen denen sich der Sensor befindet. Bei dem Dehnungssensor handelt es sich vorteilhafterweise um wenigstens einen Dehnungsmessstreifen, ein elektroaktives Polymer und/oder ein Faser-Bragg-Element.

Unter einem Dehnungsmessstreifen wird insbesondere ein Sensor verstanden, bei dem sich der elektrische Widerstand beispielsweise innerhalb einer metallischen Leiterbahn, ändert, sobald der Dehnungsmessstreifen einer Dehnung ausgesetzt wird. Aus der Änderung des elektrischen Widerstandes lassen sich Rückschlüsse auf die Änderung der Dehnung und damit auf den Abstand zwischen den beiden Punkten an den Enden des Dehnungsmessstreifens schließen. Ein elektroaktives Polymer, das oftmals auch als künstlicher Muskel bezeichnet wird, verändert seine Form und insbesondere seine Länge, wenn eine elektrische Spannung angelegt wird. Ein solches elektroaktives Polymer lässt sich jedoch umgekehrt auch als Sensor für eine Längenänderung verwenden, da die Längenänderung des elektroaktiven Polymers eine elektrische Spannung verursacht. Da auch hier der Zusammenhang bekannt ist, kann aus der detektierbaren elektrischen Spannung auf die Veränderung der Länge des Polymers und damit die Änderung des Abstandes zwischen seinen beiden Enden geschlossen werden.

Ein Faser-Bragg-Element hingegen ist ein optisches Bauteil, bei dem ein Lichtleiter verwendet wird, in dessen Kern ein Material eingebracht ist, das über variierende Brechungsindizes verfügt. Es handelt sich um sogenannte optische eingeschriebene Interferenzfilter. Auch diese Elemente sind dehnungssensitiv, da die sogenannte Bragg-Wellenlänge, die der Mittenwellenlänge der Filterbandbreite entspricht, von einer mechanischen Dehnung oder mechanischen Spannung abhängt. Auch hier kann über den bekannten Zusammenhang auf eine Änderung der Dehnung und damit auf eine Änderung des Abstandes zurückgeschlossen werden.

In einer bevorzugten Ausgestaltung verfügt der Hüllkörper über eine Vielzahl, bevorzugt wenigstens 10, weiter bevorzugt wenigstens 20, besonders bevorzugt wenigstens 50 oder 100 Dehnungssensoren, die im Material des Grundkörpers des Hüllkörper so angeordnet sind, dass sie vorzugsweise äquidistant, über die Gliedmaße verteilt sind, sobald der Hüllkörper über die Gliedmaße gezogen wurde. Auch auf diese Weise lassen sich aufgrund der bekannten Nachbarschaftsbeziehungen zwischen unterschiedlichen Endpunkten der jeweiligen Dehnungssensoren aus den veränderten Längen der Dehnungssensoren Rückschlüsse auf die Kontur des Hüllkörpers treffen, sobald der Hüllkörper über die Gliedmaße gezogen wurde.

Vorteilhafterweise ist wenigstens einer der Sensoren ein Formsensor. Unter einem Formsensor wird im Rahmen der vorliegenden Erfindung ein Sensor verstanden, der eine Längenausdehnung aufweist, und in der Lage ist, über entsprechende Messdaten nicht nur Aussagen über seine Länge, sondern auch über seine geometrische Form zu treffen. Besonders bevorzugte Beispiele sind Sensoren, die beispielsweise mehrere Lagen aus Kunststoffen aufweisen, die in einer Sandwichstruktur angeordnet sind. Kommt es zu einer Veränderung der Kontur eines derartigen Kontursensors beispielsweise in Form von Biegungen, werden die einzelnen Schichten unterschiedlich stark gedehnt oder gestaucht, was beispielsweise zu einer Veränderung der Dicke des Lagenpakets des Sensors führt. Diese wird an verschiedenen Stellen gemessen, so dass verschiedene Biegungen bestimmt werden können. Alternativ oder zusätzlich dazu können sogenannte kabelartige Formsensoren verwendet werden, wie sie beispielsweise von der Firma TST-inno angeboten werden. Alternativ oder zusätzlich sind auch Lichtleiter, die nach dem Faser-Bragg-Prinzip mit hinreichend vielen Stützstellen versehen sind, als Formsensoren verwendbar. Dabei sind wenigstens drei Stützstellen ausreichend, mehr jedoch von Vorteil. Je mehr Stützstellen vorhanden und je dichter diese angeordnet sind, desto genauer ist die Formdetektion des so ausgestalteten Formsensors.

Vorzugsweise verfügt der Hüllkörper über eine Kommunikationsschnittstelle, durch die die von dem wenigstens einen Sensor erfassten Messdaten an eine elektronische Datenverarbeitungseinrichtung, insbesondere einen Mikroprozessor übermittelbar sind. Dort können dann aus den Messdaten die gewünschten Informationen über die Kontur des Hüllkörpers erfasst werden.

In einer bevorzugten Ausgestaltung befindet sich eine entsprechende elektronische Datenverarbeitungseinrichtung bereits im oder am Grundkörper des Hüllkörper, insbesondere am distalen Ende. Auf diese Weise ist zur Erfassung der Kontur des Hüllkörper und damit der Kontur der Gliedmaßen dem Hüllkörper lediglich der Hüllkörper nötig, ohne dass zusätzliche Bauteile und/oder andere Geräte verwendet werden müssen. Dadurch wird der apparative Aufwand zur Ermittlung der Kontur weiter verringert.

Vorzugsweise ist der Grundkörper aus einem elastischen Material hergestellt. Erfindungsgemäß handelt es sich bei dem Hüllkörper um einen Prothesenliner, vorzugsweise für eine Beinprothese, Unterschenkelprothese oder eine Oberschenkelprothese, wobei der Grundkörper vorzugsweise aus einem Linermaterial hergestellt ist. Linermaterialien sind insbesondere Silikon, Polyurethan oder TPE, wobei diese Materialien einzeln oder im Verbund miteinander und/oder im Verbund mit einem Textil und/oder einer Oberflächenbeschichtung das Linermaterial bilden können.

Alternativ zu dem Prothesenliner kann der Hüllkörper auch eine Bandage sein. Mittels einer solchen Bandage können durch alle Verfahren, bei denen bereits heute optische Scanner eingesetzt werden, 3D-Modelle und 3D-Muster für spätere Fertigungsverfahren, beispielsweise CAM (Computer Aided Manufacturing), erstellt werden. Dazu zählen Fräsverfahren ebenso wie additive Verfahren, beispielsweise 3D-Druckverfahren. Auch für diagnostische Zwecke, beispielsweise den Vergleich zweier unterschiedlicher Zustände sind durch Bandage erfasste Konturen und Formen verwendbar.

Die Erfindung löst die gestellte Aufgabe zudem durch ein Verfahren zum zumindest teilweisen Erfassen einer Kontur einer Gliedmaße wobei das Verfahren die folgenden Schritte aufweist:
- Anlegen eines Hüllkörpers der hier beschriebenen Art an die Gliedmaße,
- Erfassen von Messdaten mittels des wenigstens einen Sensors,
- Ermitteln von Abständen und/oder Relativpositionen zwischen zwei Punkten in oder an dem Grundkörper in einer elektronischen Datenverarbeitungseinrichtung.

Bevorzugt wird die Form der Gliedmaße während zumindest eines Teilvorganges des Erfassens der Messdaten manipuliert. Dies kann händisch oder durch eine Vorrichtung erfolgen.

Besonders bevorzugt werden Abstände und/oder Relativpositionen zwischen wenigstens 10 Punkten, bevorzugt wenigstens 20 Punkten, weiter bevorzugt wenigstens 50 Punkten und besonders bevorzugt wenigstens 100 Punkten ermittelt. Je mehr Abstände und/oder Relativpositionen ermittelt werden können, desto genauer ist das Bild, das von der Kontur der Gliedmaße entsteht. Die einzelnen Sensoren können dabei in Form eines Bus-Systems, in Serie oder Reihe geschaltet oder in sonstiger Weise miteinander verbunden werden. Je nach Sensoren können die einzelnen Sensoren gleichzeitig, nacheinander in einer bestimmten Reihenfolge, zyklisch oder in sonstiger Weise angesteuert werden.

Vorzugsweise werden kontinuierliche Messdaten erfasst. Alternativ kann das Erfassen der Messdaten ausgelöst werden, wenn der Hüllkörper von außen verformt wird, also insbesondere ein Druck von außen auf zumindest einen Teil des Hüllkörpers wirkt.

Mit Hilfe der beiliegenden Zeichnungen werden nachfolgend einige Ausführungsbeispiele der vorliegenden Erfindung näher erläutert. Es zeigen:
- Figuren 1 bis 4 -: verschiedene Ausführungsformen eines Hüllkörpers gemäß einem Ausführungsbeispiel der Erfindung als Prothesenliner und
- Figuren 5 bis 8 -: verschiedene Ausführungsformen eines Hüllkörpers gemäß einem Ausführungsbeispiel der Erfindung als Bandage.

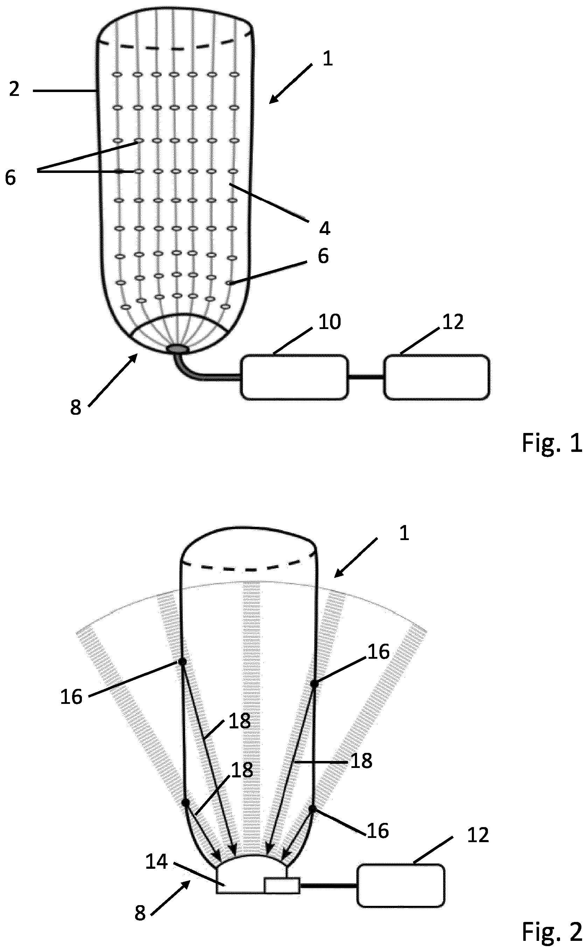

Figur 1 zeigt einen Hüllkörper 1 gemäß einem ersten Ausführungsbeispiel der vorliegenden Erfindung in Form eines Prothesenliners. Er verfügt über einen Grundkörper 2 aus einem Linermaterial. In dem Linermaterial befinden sich mehrere Glasfasern 4, in die Faser-Bragg-Sensoren 6 eingearbeitet sind. Die Glasfasern 4 laufen an einem distalen Ende 8 des Liners zusammen und können von einem Interrogator 10 mit Licht beaufschlagt werden. Dies geschieht vorzugsweise nacheinander. Die einzelnen Faser-Bragg-Sensoren 6 verfügen über ein charakteristisches Verhalten als optische Interferenzfilter. Sie absorbieren Licht bestimmter Wellenlänge, wobei diese Wellenlänge, wie bereits dargelegt, von der Dehnung oder der mechanischen Spannung des Faser-Bragg-Sensors 6 abhängt. Insbesondere für den Fall, dass die einzelnen Faser-Bragg-Sensoren 6, die durch eine einzige Glasfaser 4 miteinander verbunden sind, unterschiedlich ausgebildet werden und unterschiedliche Absorptionsspektren aufweisen, können diese nacheinander oder sogar gleichzeitig abgefragt werden. Dazu ist es beispielsweise von Vorteil, wenn die Faser-Bragg-Sensoren 6, die dem Interrogator 10 näher sind, elektromagnetische Strahlung nicht absorbieren, die von Faser-Bragg-Sensoren 6, die durch die gleiche Glasfaser 4 verbunden aber vom Interrogator 10 weiter entfernt sind, absorbieren. Sind die Faser-Bragg-Sensoren einer mechanischen Spannung oder Dehnung ausgesetzt, verschiebt sich insbesondere die Mittenfrequenz, die auch als Bragg-Frequenz bekannt ist, in bekannter Weise, so dass auf die Dehnung zurückgeschlossen werden kann. Da sich die Reihenfolge der einzelnen Faser-Bragg-Sensoren 6 entlang der Glasfasern 4 nicht ändern kann, kann aus den unterschiedlichen Dehnungen auch auf die Kontur eines Amputationsstumpfes zurückgeschlossen werden, der sich innerhalb des Prothesenliners befindet, in Figur 1 jedoch nicht dargestellt ist.

Der Interrogator 10 fragt die einzelnen Faser-Bragg-Sensoren 6 ab und übergibt die Messdaten an eine elektronische Datenverarbeitungseinrichtung 12, in der die eigentliche Auswertung stattfindet.

Figur 2 zeigt eine andere Ausgestaltung des Prothesenliners 1. Im Bereich des distalen Endes 8 befindet sich ein Sender 14, der eine Messstrahlung aussendet. Im gezeigten Ausführungsbeispiel handelt es sich dabei um Ultraschallwellen. Diese werden von Reflektoren 16 reflektiert und gelangen entlang der Pfeile 18 zurück zum Sensor, der auch einen Empfänger beinhaltet. Die Messdaten werden dann an die elektronische Datenverarbeitungseinrichtung 12 übergeben und ausgewertet.

Der Vorteil von Ultraschallwellen liegt insbesondere darin, dass diese durch den Sender 14 in das Weichteilgewebe des Amputationsstumpfes eingekoppelt werden und sich in diesem ausdehnen. Es ist nicht notwendig, dafür zu sorgen, dass sich die Ultraschallwellen durch das Linermaterial, das beispielsweise ein elastischer Silikonwerkstoff sein kann, ausbreiten. Der Sender 14 kann beispielweise im Dauerbetrieb senden oder in Form von Pulsen seine Messstrahlung abgegeben. Dabei kann auch ein rotierender Sender 14 verwendet werden, wobei nicht zwangsläufig der Sender 14 selbst rotierenden muss, sondern lediglich die ausgesandte Messstrahlung in unterschiedliche Winkelbereiche, die vorzugsweise rotieren, abgegeben wird. Die Messstrahlung, im vorliegenden Ausführungsbeispiel also die Ultraschallstrahlung, trifft auf die Reflektoren 16 und wird von diesen zurück reflektiert und erreicht den Empfänger, der in Figur 2 nicht gesondert dargestellt ist. Über Laufzeitmessungen können auf diese Weise die Abstände der einzelnen Reflektoren 16 vom Sender 14 bestimmt werden. Nachteilig ist jedoch, dass insbesondere Ultraschallsignale an Grenzflächen reflektiert werden können und auch knochige Bestandteile eines Amputationsstumpfes ein Signal liefern.

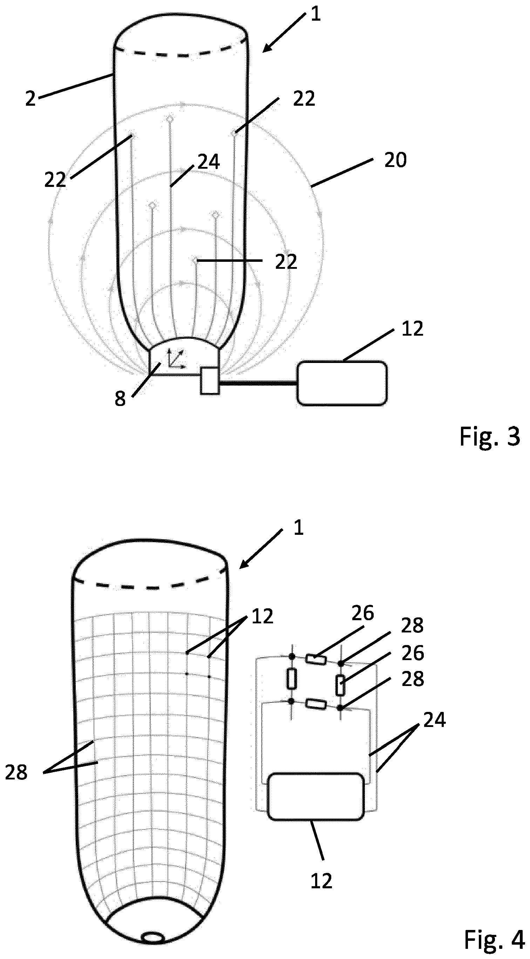

Figur 3 zeigt eine weitere Ausführungsform des Prothesenliners 1. Im Bereich des distalen Endes 8 befindet sich eine Sendespule, die mit Wechselstrom beaufschlagt wird und ein magnetisches Wechselfeld 20 aussendet. Auch dieses ist vorzugsweise rotierend ausgebildet, so dass sich auch die Hauptsenderichtung des Senders 14 verändert. Im Innern des Linermaterials des Grundkörpers 2 des Prothesenliners befinden sich Empfänger 22 in Form von Empfangsspulen, die über elektrische Leitungen 24 mit dem distalen Ende 8 verbunden sind. Das magnetische Wechselfeld 20 induziert in den Empfängern 22 einen elektrischen Strom und eine elektrische Spannung, die über die elektrische Leitung 24 abgegriffen und gemessen werden kann. Die Größe und Phase des elektrischen Stroms und/oder der elektrischen Spannung, die in den Empfängern 22 induziert wird, hängt sowohl vom Abstand der Empfänger 22 vom Sender 14 als auch von der Orientierung und der Richtung des Empfängers 22 relativ zum Sender 14 ab. Diese Messdaten werden an die elektronische Datenverarbeitungseinrichtung 12 übergeben und dort ausgewertet.

Figur 4 zeigt ein weiteres Ausführungsbeispiel des Prothesenliners 1, in dem in Form eines Netzes Dehnungssensoren in Form von Dehnungsmessstreifen 26 angeordnet sind. Dabei bilden sie im gezeigten Ausführungsbeispiel ein Quadratgitter mit Kreuzungspunkten 28, wobei sich zwischen jeweils zwei Kreuzungspunkten 28 ein Dehnungsmessstreifen 26 befindet. Diese Anordnung ist vergrößert im rechten Bereich der Figur 4 dargestellt. Wird nun der Prothesenliner über einen Amputationsstumpf gezogen, dehnt er sich der Form des Amputationsstumpfes entsprechend an einigen Stellen mehr als anderen. Durch elektrische Leitungen 24, die im rechten Teil der Figur 4 dargestellt sind, sind die einzelnen Kreuzungspunkte 28 mit elektrischem Strom und/oder elektrischer Spannung beaufschlagbar. Eine elektronische Datenverarbeitungseinrichtung 12 steuert diesen Prozess. Durch Anlegen einer elektrischen Spannung wird der Dehnungsmessstreifen 26, der zwischen den beiden angesprochenen Kreuzungspunkten 28 liegt, ausgemessen und die so erhaltenen Messsignale werden von der elektronischen Datenverarbeitungseinrichtung 12 ausgewertet. Da die Nachbarschaftsverhältnisse der unterschiedlichen Dehnungsmessstreifen 26 und auch der Kreuzungspunkt 28 bekannt sind und sich nicht ändern können, kann aus der Kenntnis der jeweiligen Strecken zwischen zwei benachbarten Kreuzungspunkten 28 ein detailliertes Bild über die Kontur des Prothesenliners ermittelt werden. Je dichter und engmaschiger das Netz ist, desto detaillierter und besser ist das Abbild der Kontur.

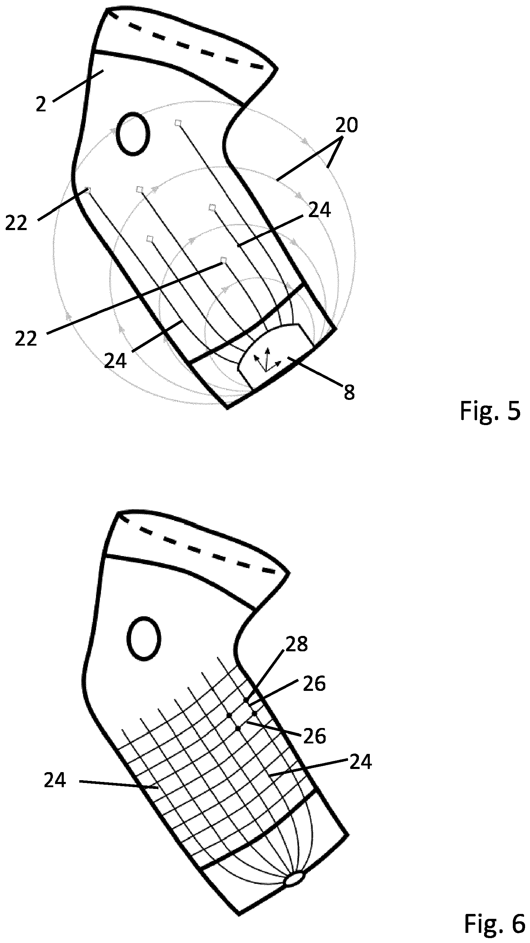

Die Figuren 5 bis 8 zeigen unterschiedliche Ausführungsformen des Hüllkörpers 1 in Form einer Bandage. Dabei entspricht die Funktionsweise der Bandage gemäß Figur 5 der des Prothesenliners gemäß Figur 3. Im distalen Bereich ist wieder die Sendespule dargestellt, die das magnetische Wechselfeld 20 aussendet. Im Inneren des Grundkörpers 2 befinden sich wieder Empfänger 22, die über elektrische Leitungen 24 mit einem distalen Ende der Bandage verbunden sind. Das magnetische Wechselfeld 20 induziert in den Empfängern 22 einen elektrischen Strom und eine elektrische Spannung, die über die elektrische Leitung 24 abgegriffen und gemessen werden kann.

Die Funktionsweise der Bandage gemäß Figur 6 entspricht der des Liners gemäß Figur 4. Auch die Bandage gemäß Figur 6 verfügt über ein Netz von Dehnungsmessstreifen 26, die zwischen Kreuzungspunkten 28 angeordnet sind. Durch elektrische Leitungen 24, die sich in den Kreuzungspunkten kreuzen, können die einzelnen Dehnungsmessstreifen 26 mit elektrischem Strom oder elektrischer Spannung beaufschlagt werden.

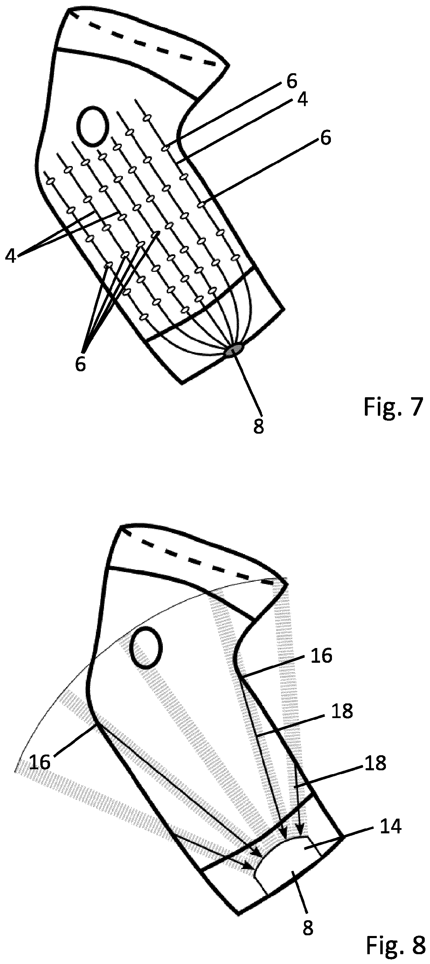

Die Funktionsweise der Bandage gemäß Figur 7 entspricht der Funktionsweise des Prothesenliners gemäß Figur 1. Faser-Bragg-Sensoren 6 sind über Glasfasern 4 miteinander verbunden, die an einem distalen Ende 8 zusammengeführt werden.

Die Funktionsweise der Bandage gemäß Figur 8 entspricht der des Liners aus Figur 2. Im Bereich des distalen Endes 8 befindet sich der Sender 14, der die Messstrahlung aussendet, die im gezeigten Ausführungsbeispiel beispielsweise Ultraschallwellen sein können. Diese werden von Reflektoren 16, die im gezeigten Ausführungsbeispiel Grenzflächen zwischen zwei unterschiedlichen Materialien darstellen, entlang der Pfeile 18 reflektiert und zum Sender zurückbefördert. Da der Sender auch einen Empfänger beinhaltet, können so Messdaten ausgewertet und übergeben werden.

### Bezugszeichenliste

- 1: Hüllkörper
- 2: Grundkörper
- 4: Glasfaser
- 6: Faser-Bragg-Sensor
- 8: distales Ende
- 10: Interrogator
- 12: elektronische Datenverarbeitungseinrichtung
- 14: Sender
- 16: Reflektor
- 18: Pfeil
- 20: magnetisches Wechselfeld
- 22: Empfänger
- 24: elektrische Leitung
- 26: Dehnungsmessstreifen
- 28: Kreuzungspunkt

## Patentansprüche

1. Hüllkörper (1) zum zumindest teilweisen Erfassen einer Kontur einer Gliedmaße, wobei der Hüllkörper (1) einen Grundkörper (2) aufweist, wobei der Hüllkörper (1) wenigstens einen Sensor (6) aufweist, der eingerichtet ist, Messdaten zu erfassen, aus denen ein Abstand und/oder eine Relativposition zwischen zwei Punkten in oder an dem Grundkörper (2) ermittelbar ist, **dadurch gekennzeichnet, dass** der Hüllkörper (1) ein Prothesenliner ist und der Grundkörper (2) aus einem Linermaterial hergestellt ist.

2. Hüllkörper (1) nach Anspruch 1, **dadurch gekennzeichnet, dass** der Sensor (6) einen Sender (14) für eine Messstrahlung und einem Empfänger (22) für die Messstrahlung aufweist, die derart angeordnet sind, das vom Sender (14) ausgesandte Messstrahlung zumindest teilweise vom Empfänger (22) empfangen wird.

3. Hüllkörper (1) nach Anspruch 2, **dadurch gekennzeichnet, dass** sich am ersten Punkt ein Sender (14) und ein Empfänger (22) befindet, wobei sich vorzugsweise am zweiten Punkt ein Reflektor (16) für die Messstrahlung befinde, oder dass sich der Sender (14) am ersten Punkt und der Empfänger (22) am zweiten Punkt befindet.

4. Hüllkörper (1) nach Anspruch 3, **dadurch gekennzeichnet, dass** die Messstrahlung elektromagnetische Strahlung, insbesondere sichtbares Licht, Radarstrahlung und/oder Röntgenstrahlung, magnetische Strahlung beispielsweise in Form eines magnetischen Wechselfeldes und/oder Schallwellen, insbesondere Ultraschall, aufweist.

5. Hüllkörper (1) nach Anspruch 3 oder 4, **dadurch gekennzeichnet, dass** der Hüllkörper (1) mehrere Empfänger (22) und/oder mehrere Reflektoren (16) aufweist.

6. Hüllkörper (1) nach einem der vorstehenden Ansprüche, **dadurch gekennzeichnet, dass** der wenigstens eine Sensor (6) einen Dehnungssensor, insbesondere einen Dehnungsmessstreifen (26), ein elektroaktives Polymer und/oder ein Faser-Bragg-Element aufweist

7. Hüllkörper (1) nach einem der vorstehenden Ansprüche, **dadurch gekennzeichnet, dass** wenigstens einer der Sensoren (6) ein Formsensor ist.

8. Hüllkörper (1) nach einem der vorstehenden Ansprüche, **dadurch gekennzeichnet, dass** der Hüllkörper (1) einer Kommunikationsschnittstelle aufweist, durch die die von dem wenigstens einen Sensor erfassten Messdaten an eine elektronische Datenverarbeitungseinrichtung (12), insbesondere einen Mikroprozessor, übermittelbar sind.

9. Hüllkörper (1) nach einem der vorstehenden Ansprüche, **dadurch gekennzeichnet, dass** der Grundkörper (2) aus einem elastischen Material hergestellt ist.

10. Verfahren zum zumindest teilweisen Erfassen einer Kontur einer Gliedmaße, wobei das Verfahren die folgenden Schritte aufweist:
- Anlegen eines Hüllkörpers (1) nach einem der vorstehenden Ansprüche an die Gliedmaße,
- Erfassen von Messdaten mittels des wenigstens einen Sensors (6),
- Ermitteln von Abständen und/oder Relativpositionen zwischen zwei Punkten in oder an dem Grundkörper (2) in einer elektronischen Datenverarbeitungseinrichtung (12).

11. Verfahren nach Anspruch 10, **dadurch gekennzeichnet, dass** eine Manipulation einer Form der Gliedmaße während zumindest eines Teilvorganges des Erfassens der Messdaten erfolgt.

12. Verfahren nach Anspruch 10 oder 11, **dadurch gekennzeichnet, dass** Abstände und/oder Relativpositionen zwischen wenigstens 10 Punkten, bevorzugt wenigstens 20 Punkten, weiter bevorzugt wenigstens 50 Punkten, besonders bevorzugt wenigstens 100 Punkten ermittelt werden.

13. Verfahren nach einem der Ansprüche 10 bis 12, **dadurch gekennzeichnet, dass** das Erfassen der Messdaten kontinuierlich erfolgt oder automatisch ausgelöst wird, wenn der Hüllkörper von außen verformt wird.

## Claims

1. An enveloping body (1) for at least partially recording a contour of a limb, wherein the enveloping body (1) has a base body (2), wherein the enveloping body (1) has at least one sensor (6) that is configured to record measurement data which can be used to determine a distance and/or relative position between two points in or on the base body (2) **characterized in that** the enveloping body (1) is a prosthetic liner and the base body is made of a liner material.

2. The enveloping body (1) according to claim 1, **characterized in that** the sensor (6) has a transmitter (14) for a measuring radiation and a receiver (22) for the measuring radiation, which are arranged in such a way that measuring radiation emitted by the transmitter (14) is at least partially received by the receiver (22).

3. The enveloping body (1) according to claim 2, **characterized in that** a transmitter (14) and a receiver (22) are located at the first point, wherein a reflector (16) for the measuring radiation is preferably located at the second point, or that the transmitter (14) is located at the first point and the receiver (22) at the second point.

4. The enveloping body (1) according to claim 3, **characterized in that** the measuring radiation is electromagnetic radiation, in particular visible light, radar radiation and/or X-rays, magnetic radiation, for example in the form of an alternating magnetic field and/or sonic waves, in particular ultrasonic waves.

5. The enveloping device (1) according to claim 3 or 4, **characterized in that** the enveloping device (1) comprises several receivers (22) and/or several reflectors (16).

6. The enveloping device (1) according to one of the preceding claims, **characterized in that** the at least one sensor (6) comprises a strain sensor, in particular a strain gauge (26), an electroactive polymer and/or a fiber Bragg element.

7. The enveloping device (1) according to one of the preceding claims, **characterized in that** at least one of the sensors (6) is a shape sensor.

8. The enveloping device (1) according to one of the preceding claims, **characterized in that** the enveloping body (1) features a communication interface by means of which the measurement data recorded by the at least one sensor can be transmitted to an electronic data processing device (12), in particular a micro-processor.

9. The enveloping device (1) according to one of the preceding claims, **characterized in that** the base body (2) is made from an elastic material.

10. A method for at least partially recording a contour of a limb, wherein the method comprises the following steps:
- mounting an enveloping body (1) according to one of the preceding claims on the limb,
- recording measurement data by means of the at least one sensor (6),
- detecting distances and/or relative positions between two points in or on the base body (2) in an electronic data processing device (12).

11. The method according to claim 10, **characterized in that** a manipulation of a shape of the limb occurs during at least part of the process of recording the measurement data.

12. The method according to claim 10 or 11, **characterized in that** distances and/or relative positions between at least 10 points, preferably at least 20 points, preferably at least 50 points, especially preferably at least 100 points are detected.

13. The method according to one of the claims 10 to 12, **characterized in that** the recording of the measurement data is continuous or triggered automatically when the enveloping body is deformed from the outside.

## Revendications

1. Corps enveloppant (1) destiné à saisir au moins partiellement un contour d'un membre, le corps enveloppant (1) présentant un corps de base (2), le corps enveloppant (1) présentant au moins un capteur (6) qui est conçu pour saisir des données de mesure à partir desquelles une distance et/ou une position relative entre deux points dans ou sur le corps de base (2) peut être déterminée,
**caractérisé en ce que** le corps enveloppant (1) est une doublure prothétique, et le corps de base (2) est fabriqué en un matériau de la doublure.

2. Corps enveloppant (1) selon la revendication 1,
**caractérisé en ce que** le capteur (6) présente un émetteur (14) pour un rayonnement de mesure et un récepteur (22) pour le rayonnement de mesure, qui sont disposés de telle sorte que le rayonnement de mesure émis par l'émetteur (14) est au moins partiellement reçu par le récepteur (22).

3. Corps enveloppant (1) selon la revendication 2,
**caractérisé en ce qu'**un émetteur (14) et un récepteur (22) se trouvent au premier point, de préférence, un réflecteur (16) pour le rayonnement de mesure se trouvant au deuxième point, ou
**en ce que** l'émetteur (14) se trouve au premier point et le récepteur (22) se trouve au deuxième point.

4. Corps enveloppant (1) selon la revendication 3,
**caractérisé en ce que** le rayonnement de mesure inclut le rayonnement électromagnétique, en particulier la lumière visible, le rayonnement radar et/ou les rayons X, le rayonnement magnétique, par exemple sous la forme d'un champ magnétique alternatif, et/ou les ondes sonores, en particulier les ultrasons.

5. Corps enveloppant (1) selon la revendication 3 ou 4,
**caractérisé en ce que** le corps enveloppant (1) comporte plusieurs récepteurs (22) et/ou plusieurs réflecteurs (16).

6. Corps enveloppant (1) selon l'une des revendications précédentes,
**caractérisé en ce que** ledit au moins un capteur (6) comprend un capteur de contrainte, en particulier une jauge de contrainte (26), un polymère électroactif et/ou un élément de Bragg à fibres.

7. Corps enveloppant (1) selon l'une des revendications précédentes,
**caractérisé en ce que** l'un au moins des capteurs (6) est un capteur de forme.

8. Corps enveloppant (1) selon l'une des revendications précédentes,
**caractérisé en ce que** le corps enveloppant (1) présente une interface de communication permettant de transmettre les données de mesure, saisies par ledit au moins un capteur, à un dispositif électronique de traitement de données (12), en particulier à un microprocesseur.

9. Corps enveloppant (1) selon l'une des revendications précédentes,
**caractérisé en ce que** le corps de base (2) est fabriqué en un matériau élastique.

10. Procédé pour saisir au moins partiellement un contour d'un membre, le procédé comprenant les étapes suivantes consistant à :
- appliquer un corps enveloppant (1) selon l'une des revendications précédentes sur le membre,
- saisir des données de mesure au moyen dudit au moins un capteur (6),
- déterminer des distances et/ou des positions relatives entre deux points dans ou sur le corps de base (2) dans un dispositif électronique de traitement de données (12).

11. Procédé selon la revendication 10,
**caractérisé en ce qu'**une manipulation d'une forme du membre est effectuée pendant au moins une opération partielle de la saisie des données de mesure.

12. Procédé selon la revendication 10 ou 11,
**caractérisé en ce que** des distances et/ou des positions relatives entre au moins 10 points, de préférence au moins 20 points, de préférence encore au moins 50 points, de manière particulièrement préférée au moins 100 points sont déterminées.

13. Procédé selon l'une des revendications 10 à 12,
**caractérisé en ce que** la saisie des données de mesure est effectuée en continu ou est déclenchée automatiquement lorsque le corps enveloppant est déformé de l'extérieur.
